# EUROPEAN PATENT APPLICATION

(11) **EP 0 583 520 A1**
(43) Date of publication of application: **23.02.1994**
(21) Application number: 92307551.9
(22) Date of filing: 18.08.1992
(51) Int. Cl.: A61B 17/58

(54) **Device for treating funnel chest**

(71) Applicant: NAUCHNO-PROIZVODSTENNOE OBIEDINENIE " EKRAN", Moscow 3 (RU); TSENTRALNY NAUCHNO-ISSLEDOVATELSKY INSTITUT TRAVMATOLOGII I ORTOPEDII IMENI N.N. PRIOROVA, Moscow (RU)
(72) Inventor: Shaposhnikov, July Georgievich, Moscow (SU); Malakhov, Oleg Alexeevich, Moscow (SU); Kornachev, Alexei Lvovich, Moscow (SU); Belykh, Sergei Ivanovich, Moscow (SU); Davydov, Anatoly Borisovich, Moscow (SU); Berentsveig, Boris Ruvinovich, Moscow (SU)
(74) Representative: Lerwill, John

(57) **Abstract**

Holder (1) is in the form of a planar plate having rod-like members (4, 5) protruding from it in opposite directions and bent in a plane drawn at right angles to the base of the plate. The planar plate is designed for attachment to the body of the breast bone and the rod-like members are designed for attachment to the ribs symmetrical with respect to the breast bone.

## Description

The invention relates to the medicine, and in particular, it deals with treatment of funnel chest.

It is known to use an osteal autoplastic graft out of the tibia a patient which is positioned in the zone of longitudinal osteotomy of the body of the breast bone to treat funnel deformity of the chest (SU,A,489505).

The use of the autoplastic graft does not allow the shape of the chest to be brought completely to its regular configuration and results in inevitable resorption of the autoplastic graft so as to impair the holding capacity. It should be added that taking the autoplastic graft calls for surgical intervention and is accompanied by serious injuries to the patient. The autoplastic graft method has not come into a widespread use for the above reasons.

It is also known to treat funnel chest with the aid of a device having a breast bone holder in the form of a metal stem of rectangular cross-section. The rod is composed of individual parts at its ends, and the branches of the stem diverge in opposite directions with respect to the longitudinal axis of the stem. The branches are attached to the ribs extending at different parallel levels on either side of the breast bone body and are secured thereto so as to hold the breast bone (SU, A, 1230592).

The method with the use of the device having such a holder is deficient in the fact that the stem should be bent by a surgeon during operation which requires application of a big effort. This is also associated with the need to move apart the branches and to fit them afterwards as the amount of bend changes. This design of the holder requires its attachment at four points at the ribs of different levels on either side of the breast bone. As a result, torques are built up which depend on the amount of deflection of the branches from the axis of the stem. This gives rise to undesired mechanical stresses in the breast bone. On the other hand, this attachment restricts subsequent growth of the chest. And finally it should be born in mind that the operation has to be repeated to remove the holder.

Therefore, the prior art holder is a source of a number of inconveniences for both a surgeon and a patient.

The invention is based on the problem of providing a holder of a device for treating funnel chest which is so constructed as to make choice of the holder to fit to ergonometric parameters of a patient for a fixed holding of the body of the breast bone and the ribs and to dispense with a repeated surgical intervention for the removal of the holder.

The above problem is solved by the fact that it is an object of the invention to provide a holder for a device for treating funnel chest which is so constructed as to fit to ergonometric parameters of a patient and to dispense with a repeated surgical intervention to remove the holder.

Another object of the invention is to facilitate operation for a surgeon during installation of the device for treating funnel chest.

Still another object of the invention is to provide an operation procedure for installation of such a new device for treating funnel chest which does not call for a repeated operation.

These and other objects are accomplished by the fact that in a device for treating funnel chest comprising a breast bone holder having fasteners, characterized by the fact that the holder is in the form of a part having a planar support surface for applying to the body of the breast bone and rod-like members protruding from this part in opposite directions and bent in a plane drawn perpendicularly with respect to the planar surface with a radius of curvature corresponding to the radius of bow of then ribs. Both the part and the rod-like member are made of biocompatible and biodegradable material.

With the use of the device for treating funnel chest having such a holder the stem need not be bent so as to substantially accelerate and facilitate operation for a surgeon. The attachment of this holder rules out appearance of torques so as to substantially lower mechanical stresses in the breast bone and ribs. Finally it should be born in mind that the holder according to the invention does not hinder the subsequent growth of the chest.

The device according to the invention is preferably made of a biocompatible and biodegradable polymeric material so as to avoid a repeated operation to remove the device for treating funnel chest.

Taking into account the manufacturing process aspect, it is preferred that the holder be in the form of a rectangular parallelepiped having rod-like members protruding from one of its sides and bent in a plane drawn perpendicularly with respect to the base of the parallelepiped. The holder may be formed by a bent stem having in its middle part a straight portion surrounded by a part having the planar support surface.

The invention will now be described in details with reference to a non-limiting embodiment of the invention illustrated in the accompanying drawings, in which:
Fig. 1 is a perspective view of a device for treating funnel chest according to the invention;
Fig. 2 is an exploded view of a device according to the invention;
Fig. 3 schematically illustrates guidelines for installation of a device according to the invention in a patient.

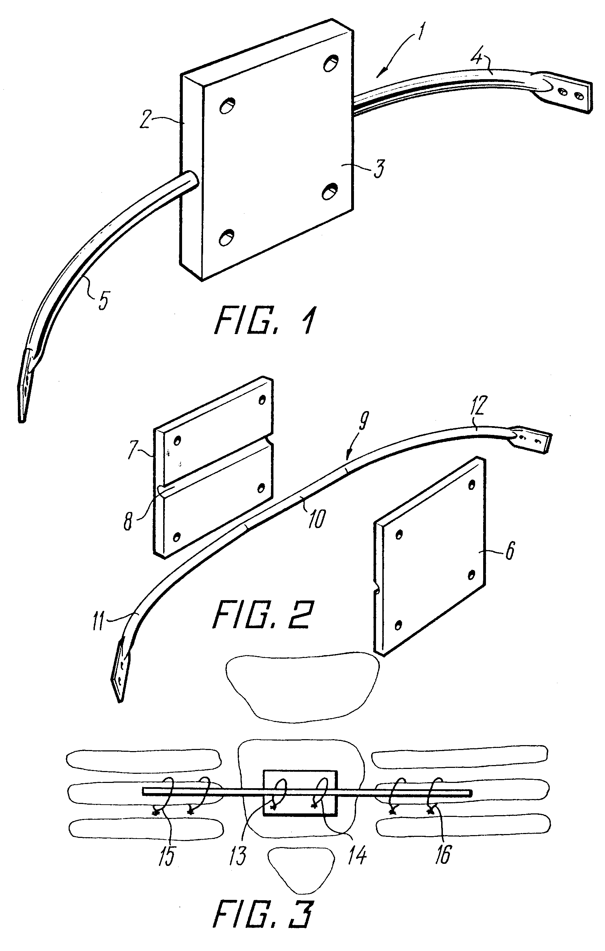

With reference to Fig. 1, a device for treating funnel chest comprises a holder 1 which is formed by a part in the form of a plate 2 shaped as a rectangular parallelepiped which has its base 3 functioning as a support surface to be placed on the body of the breast bone as will be described below. Rod-like members 4 and 5 protrude from the sides of parallelepiped 2 in opposite directions which are bent in a plane drawn perpendicularly with respect to base 3. The radius of the bend corresponds to the radius of bow of the ribs. Plate 2 and the rod-like members may be made of any biocompatible and biodegradable polymeric material. In particular, such material may be in the form of a copolymer of vinyl pyrrolidone with methyl methacrylate described in US patent No. 4,263,185 to S.I. Belykh et al., or a copolymer of vinyl pyrrolidone with butyl methacrylate described in US patent No. 5,011,493 to S.I. Belykh et al. and other materials. It will be understood that other biocompatible and biodegradable polymeric materials may be used as such polyglycolides, polylactides, polydioxanones, polycyan acrylates. It should only be born in mind that such biodegradable materials have the period of biological degrading of from 1 to 3 years. Although materials with other periods of degrading or resorption can be used as well, it should be noted that the choice of material generally depends on the fact that a material with a period of degrading of less than one year can result in a relapse of deformity of the breast bone. Of a period of resorption is longer than three years and a half, an undesired reaction of the body to a foreign object may occur. In addition, if a plate with a short period of resorption, which is less than half a year is used, reliable holding for the entire period of regeneration of the bone, which is required for ensuring the necessary strength of bond between the ribs and the breast bone, cannot be achieved.

Therefore, the choice of the device is made based on both physical and ergonometric parameters of a patient and the type of material of which the device is made.

Plate 2 of holder 1 of the breast bone may be of any shape other than rectangular parallelepiped, but at any rate holder 1 should have one planar surface designed for placing on the breastbone, the rod-like members being bent in a plane drawn perpendicularly with respect to this planar surface.

The breast bone holder of the device for treating funnel chest may be made by using various methods such as by pressing, stamping or injection molding.

Fig. 2 shows an exploded view or the holder to illustrate a simple way of making the holder. It can be seen in Fig. 2 that two halves 6 and 7 of part 2 are first made, and each half has a groove 8 in its central part. A stem 9 is also made in advance and has a straight central portion 10 and bent end portions 11 and 12 defining the protruding rod-like members. This stem is placed into groove 8 of each half 6 or 7 of part 2, and the two halves are then press-fitted to each other to obtain a device forming the holder of Fig. 1. It is preferred that a surgeon have a set of devices with various surfaces areas and radii of bend of the rods.

An operation for treating funnel chest will now be described with the use of the device according to the invention.

For making an operation, first the size of the body of the breast bone and the radius of bow of the ribs are determined. A desired set of devices according to the invention are taken with a surface area increasing and decreasing, e.g., by 5 sq. mm with respect to the measured value and with the radius of curvature also changing in the same manner with a 5 cm gradation.

The operation is carried out in the following manner.

The anterior cortical plate of the body of the breast bone is removed, and the chest is corrected. For holding, a device in which the holder has a radius of curvature corresponding to the curvature of the chest in its correct position is chosen from out of the set. The support plate is attached at two points to the breast bone body by a polyethylene terephthalate thread 13, 14, and the bent ends are attached in an inclined position to the ribs on the left and on the right from the breast bone by means of a polyethylene terephthalate thread 15, 16 at two points on either side. The wound is blind-sutured layer by layer and a tubular drain is left for 24 hours or for a longer period.

The preferred embodiment of the device for treating funnel chest was described above, and those skilled in the art can make modifications without going beyond spirit and scope of the invention. Thus the bent rod-like portions and the bent stem may be reinforced with a polyamide thread, and the material for making the plate and rods may be in the form of a mixture of a copolymer with polyethylcyan acrylate and calcium gluconate. Plate 2 may be made of any configuration other than rectangular parallelepiped such as a figure having a cross-sectional configuration of a triangle, a polygon, a circle, a trapezium, and the like. If polyhedrons are used, it is preferred that their edges are rounded.

Several devices for treating funnel chest were made with a holder provided with a rod of 5 mm in diameter and with radius of bend of 150 mm, 3 mm in diameter and with radius of bend of 110 mm, and 6 mm in diameter and with radius of bend of 180 mm. The part having the planar support surface was in the form of rectangular parallelepiped of a size of 10x10x2 mm, 12x20x2 mm, and 16x18x8mm, respectively. The use of these devices allowed a correct shape of the breast bone during the period of regeneration to be maintained.

## Claims

1. A device for treating funnel chest comprising a breast bone holder (1) having fasteners, characterized by the fact that the holder is in the form of a part (2) having a planar support surface (3) for applying to the body of the breast bone and rod-like members (4,5) protruding from this part in opposite directions and bent in a plane drawn perpendicularly with respect to the planar surface with a radius of curvature corresponding to the radius of bow of then ribs, the part having the planar surface and the rod-like member being made of biocompatible and biodegradable material.

2. A device of claim 1, characterized by the fact that the part comprises a plate in the form of a rectangular parallelepiped having rounded edges, the rod-like members protruding from its sides and being bent in a plane drawn perpendicularly with respect to the base of the parallelepiped.

3. A device of claim 1, characterized by the fact that the holder comprises a bent stem having in its middle part a straight portion (9) surrounded by the part having the planar support surface.
